# FASCICULE DE BREVET EUROPEEN

(11) **EP 0 645 986 B1**
(45) Date de publication et mention de la délivrance du brevet: **26.03.1997**
(21) Numéro de dépôt: 93913159.5
(22) Date de dépôt: 18.06.1993
(51) Int. Cl.: A61B 17/70

(54) **APPAREIL DE TRAITEMENT DU RACHIS**
APPARAT ZUR BEHANDLUNG DER WIRBELSÄULE
SPINAL THERAPY APPARATUS

(30) Priorité: 19.06.1992 FR 9207504
(43) Date de publication de la demande: 05.04.1995
(73) Titulaire: ROUSSOULY, Pierre, 69450 Saint Cyr au Mont d'Or (FR); TAGLANG, Gilbert, 67370 Griesheim sur Souffel (FR); GROSSE, Arsène, 67400 Illkirch-Graffenstaden (FR); CHOPIN, Daniel, 62155 Merlimont (FR)
(72) Inventeur: ROUSSOULY, Pierre, 69450 Saint Cyr au Mont d'Or (FR); TAGLANG, Gilbert, 67370 Griesheim sur Souffel (FR); GROSSE, Arsène, 67400 Illkirch-Graffenstaden (FR); CHOPIN, Daniel, 62155 Merlimont (FR)
(74) Mandataire: Poncet, Jean-François
(86) Numéro de dépôt international: FR9300605
(87) Numéro de publication internationale: WO9400062

(56) Documents cités:
- EP-A- 0 383 992
- EP-A- 0 408 489
- FR-A- 2 615 095
- US-A- 4 648 388
- US-A- 4 773 402
- US-A- 4 987 892

## Description

La présente invention a pour objet un appareil de traitement d'un rachis présentant une déviation anormale, d'origine dégénérative ou traumatique.

On connaît déjà de nombreux appareils pour traiter des arthroses ou des fractures vertébrales ou pour corriger des déviations de colonne vertébrale, telles que la scoliose, la lordose et la cyphose.

Par exemple, le document US-A-4 648 388 décrit un appareil de traitement du rachis comprenant des éléments d'ancrage dans les vertèbres, une tige de solidarisation à section circulaire et à surface extérieure lisse, et des coulisseaux de liaison pour relier des éléments d'ancrage à la tige de solidarisation. Les éléments d'ancrage sont des vis comportant trois parties principales, une première partie d'extrémité à filet hélicoïdal adapté pour une pénétration et une tenue dans l'os, une partie intermédiaire cylindrique lisse de diamètre réduit, et une seconde partie d'extrémité à filet hélicoïdal adapté pour le vissage d'un écrou de serrage. Les coulisseaux de liaison comportent une partie de serrage conformée pour entourer un tronçon de la tige de solidarisation, et une partie de liaison dépassant latéralement et percée de deux trous correspondants destinés à être traversés par la vis d'ancrage. On visse tout d'abord la vis d'ancrage dans l'os, on adapte ensuite le coulisseau de liaison sur la partie intermédiaire cylindrique de la vis d'ancrage, et on visse enfin l'écrou de serrage sur la seconde partie filetée de la vis de serrage pour plaquer le coulisseau de liaison contre une vertèbre et pour serrer simultanément le coulisseau de liaison autour de la tige de solidarisation.

Un tel appareil est destiné à assurer le maintien du rachis selon une courbure appropriée. Il s'avère toutefois que le maintien mécanique assuré par cet appareil n'est pas suffisant. En particulier, l'appui du coulisseau directement sur une vertèbre exclut toute possibilité de serrage efficace, par suite de la faible résistance mécanique à la compression de la vertèbre. En outre, la pose de l'appareil sur un rachis nécessite parfois l'usage simultané de deux outils, notamment pour empêcher la rotation de la vis lors du vissage ou du dévissage de l'écrou. Des entailles ménagées le long de la tige de solidarisation réduisent sa résistance mécanique, et augmentent sa flexibilité. Egalement, cet appareil n'est pas adapté à la réduction tridimensionnelle de la partie supérieure du rachis, partie dans laquelle l'implantation de vis est exclue. Et surtout, l'effort de serrage de l'écrou augmente sensiblement la contrainte de traction exercée sur la vis, en s'ajoutant aux efforts de tenue de la tige de solidarisation, ce qui réduit d'autant la résistance mécanique de l `ancrage et favorise la nécrose de l'os autour de la vis.

Le document EP-A-0 408 489 décrit un appareil permettant de relier deux vertèbres, au moyen de deux vis pédiculaires à double filetage et de deux coulisseaux reliés par une tige filetée permettant d' en régler l'écartement. La vis comporte des portées sphériques permettant un réglage d'inclinaison par rapport au coulisseau, le serrage d'un écrou de serrage sur la partie filetée de vis assurant un blocage en rotation. Ce dispositif, qui semble adapté pour le réglage d'espacement de deux vertèbres successives, est totalement inadapté pour tenir plus de deux vertèbres, et ne comporte aucun enseignement relatif au problème de l'insuffisance de tenue mécanique de tiges de liaison lisses.

### EXPOSE DE L'INVENTION

Le problème proposé par la présente invention est de concevoir une nouvelle structure d'appareil de traitement de rachis à tige de solidarisation lisse et coulisseaux de liaison pour éléments d'ancrage, qui assure une solidarisation nettement plus efficace des vertèbres et une plus grande facilité de pose et de réglage de position des éléments les uns par rapport aux autres en tridimensionnel.

L'invention résulte de l'observation selon laquelle des défauts de maintien et de positionnement des appareils connus, et simultanément une relative complexité de pose et de réglage de ces appareils, sont dus à des possibilités de pivotement soit entre les coulisseaux de liaison et la tige de solidarisation, soit entre les coulisseaux de liaison et l'élément d'ancrage correspondant. En effet, des couples de rotation importants apparaissent, lors de la réduction des déformations du rachis à traiter, et par la suite au cours du maintien du rachis traité, et les structures connues d'appareil ne permettent pas une tenue fiable de ces couples importants.

Pour cela, l'appareil de la présente invention comprend :
- des éléments d' ancrage dans les vertèbres, comportant une partie d'ancrage conformée pour s'ancrer dans ou sur l'os d'une vertèbre et prolongée par une partie cylindrique filetée sur laquelle se visse un écrou de serrage,
- une tige de solidarisation, à section circulaire et à surface extérieure lisse,
- des coulisseaux de liaison pour relier des éléments d'ancrage à la tige de solidarisation, les coulisseaux de liaison comportant une partie de serrage et une partie de liaison, la partie de serrage présentant des surfaces intérieures conformées pour entourer un tronçon de la tige de solidarisation et étant déformable pour sélectivement être serrée ou desserrée sur la tige de solidarisation, la partie de liaison comportant une première et une seconde branches se développant latéralement et parallèlement l'une à l'autre à partir de deux lèvres d'une fente longitudinale de la partie de serrage, lesdites branches de partie de liaison comportant des trous de passage correspondants pour recevoir la partie cylindrique filetée d'un élément d'ancrage permettant de serrer les branches l'une vers l'autre et de serrer ainsi la partie de serrage ;
selon l'invention :
- les éléments d'ancrage comprennent un plateau d'arrêt à face d'appui généralement perpendiculaire à l'axe de partie cylindrique filetée et se développant à partir de sa base,
- la face d'appui du plateau d'arrêt comprend des premiers reliefs antiglissement,
- la première surface de contact correspondante de première branche de partie de liaison, destinée à venir porter contre la face d'appui d'éléments d'ancrage, comprend des premiers reliefs antiglissement correspondants pour s'opposer à toute rotation et à tout déplacement latéral du coulisseau de liaison par rapport à l'élément d'ancrage après serrage de l'écrou contre la seconde surface de contact opposée de seconde branche de partie de liaison.

De préférence, les surfaces antérieures de partie de serrage comportent des seconds reliefs antiglissement, réalisant un appui discontinu qui s'oppose à la rotation du coulisseau de liaison autour de la tige de solidarisation après serrage de l'élément de serrage.

Les reliefs antiglissement s'opposent très efficacement aux rotations ou glissements pouvant apparaître à la fois entre les coulisseaux et la tige de solidarisation, et entre les coulisseaux et les éléments d'ancrage, dès le début de serrage des écrous de serrage. Simultanément, les reliefs antiglissement associés au plateau d'arrêt interdisent la rotation de l'élément d'ancrage lors du serrage de l'écrou même lorsque cet élément d'ancrage est sous forme d'une vis osseuse, de sorte qu'un seul outil suffit au serrage.

Ces dispositions permettent en outre un réglage précis de position relative des éléments les uns par rapport aux autres, notamment un réglage précis de position angulaire des coulisseaux de liaison sur la tige de solidarisation, et un réglage précis de position angulaire des coulisseaux de liaison sur les éléments d'ancrage, par un effet de verrouillage avant serrage complet.

L'appareil peut comprendre, comme élément d'ancrage, une ou plusieurs vis osseuses à double filetage de part et d'autre d'un plateau d'arrêt intermédiaire à reliefs antiglissement, un ou plusieurs crochets dont la paroi arrière sert de plateau d'arrêt et comporte les reliefs antiglissement, et éventuellement des plaques d'appui conformées pour s'adapter à la morphologie particulière de certaines zones du rachis et comportant des reliefs antiglissement.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles:
- les figures 1 et 2 sont des vues respectives de côté et de face d'un même appareil selon un mode de réalisation de l'invention ;
- la figure 3 est une vue en perspective de l'appareil des figures 1 et 2 ;
- la figure 4 est une vue en perspective d'une vis osseuse pouvant servir d'élément d'ancrage selon l'invention ;
- la figure 5 est une vue en perspective montrant un crochet pédiculaire pouvant servir d'élément d'ancrage selon l'invention ;
- la figure 6 est une vue en perspective d'un crochet lamaire pouvant servir d'élément d'ancrage selon l'invention ;
- la figure 7 est une vue en perspective d'un coulisseau de liaison selon un premier mode de réalisation de l'invention ;
- les figures 8 et 9 illustrent, respectivement en vue de côté et en vue de dessous, le coulisseau de liaison de la figure 7 ;
- la figure 10 est une vue en perspective d'un coulisseau de liaison selon un second mode de réalisation de l'invention ;
- les figures 11 et 12 illustrent, respectivement en vue de côté et en vue de dessous, le coulisseau de liaison de la figure 10 ;
- les figures 13 et 14 illustrent en perspective deux autres modes de réalisation de coulisseau de liaison selon l'invention ;
- la figure 15 est une vue en perspective d'un coulisseau de liaison double selon l'invention ;
- la figure 16 est une vue en perspective d'un coulisseau double selon un autre mode de réalisation ;
- la figure 17 est une vue en perspective d'une plaque d'appui sacré pouvant servir d'élément d'ancrage selon l'invention, et la figure 17A illustre une plaque d'appui illio-sacré similaire ;
- les figures 18 et 19 illustrent l'intérêt de la plaque d'appui sacré ;
- les figures 20 et 21 illustrent, en vue de côté et en vue de dessus, une plaque d'appui sur le corps d'une vertèbre ;
- les figures 22 et 23 illustrent, respectivement en vue de côté et en vue de dessus, une entretoise permettant de relier deux tiges de solidarisation parallèles ; et
- la figure 24 représente en perspective un crochet lamaire d' extrémité.

### DESCRIPTION DES MODES DE REALISATION PREFERES

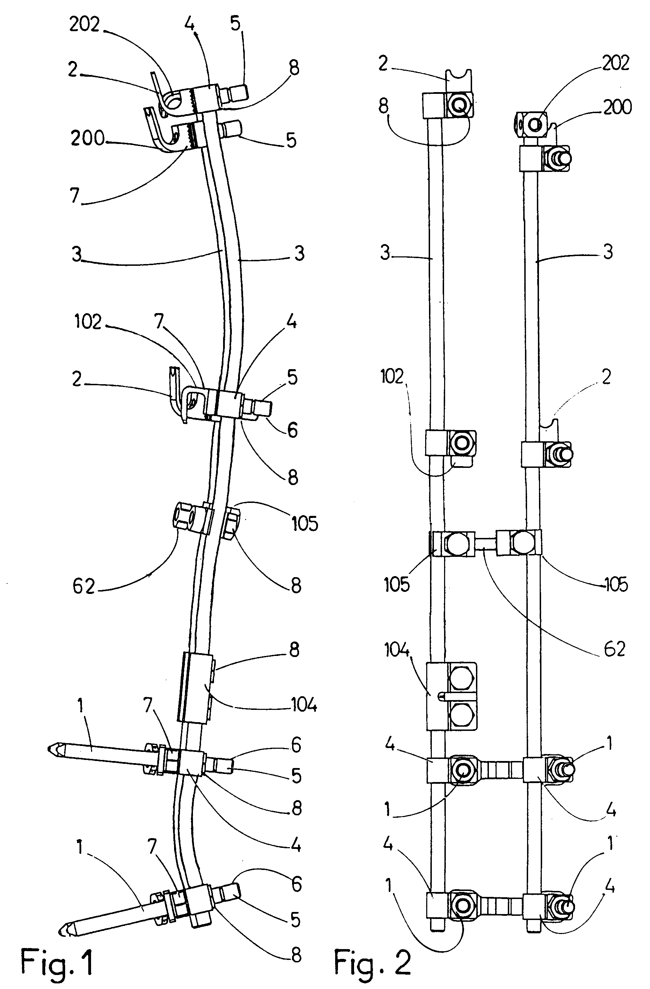

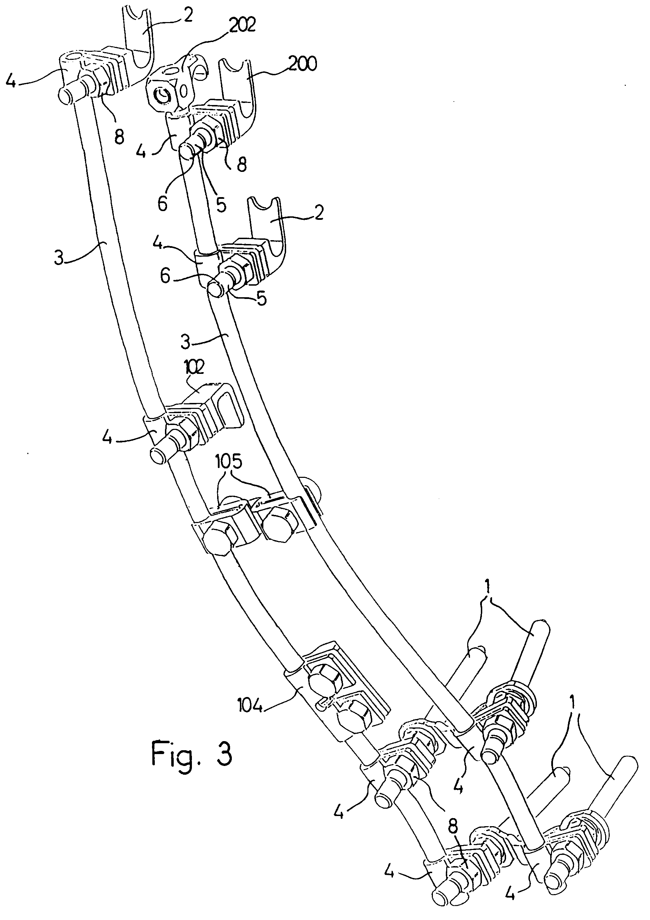

L'appareil représenté sur les figures 1 à 3 comprend des éléments d'ancrage tels que des vis osseuses 1 ou des crochets 2, 102, 202, des tiges de solidarisation 3, et des coulisseaux de liaison 4 entre chaque élément d'ancrage et la tige de solidarisation 3.

Les tiges de solidarisation 3 de l'appareil selon l'invention sont des tiges lisses, de section circulaire. On choisit le matériau et la section de manière à obtenir une élasticité optimale, adaptée aux efforts à supporter lors de l'utilisation, et de façon que la tige puisse être galbée selon la forme souhaitée pour la partie de colonne vertébrale à traiter.

Chaque élément d'ancrage, vis osseuse, crochet ou plaque d'appui, est d'une seule pièce avec une partie cylindrique filetée 5 pourvue d'un filetage extérieur 6, et comporte un plateau d'arrêt 7 limitant le serrage d'un écrou 8 sur ce filetage extérieur 6.

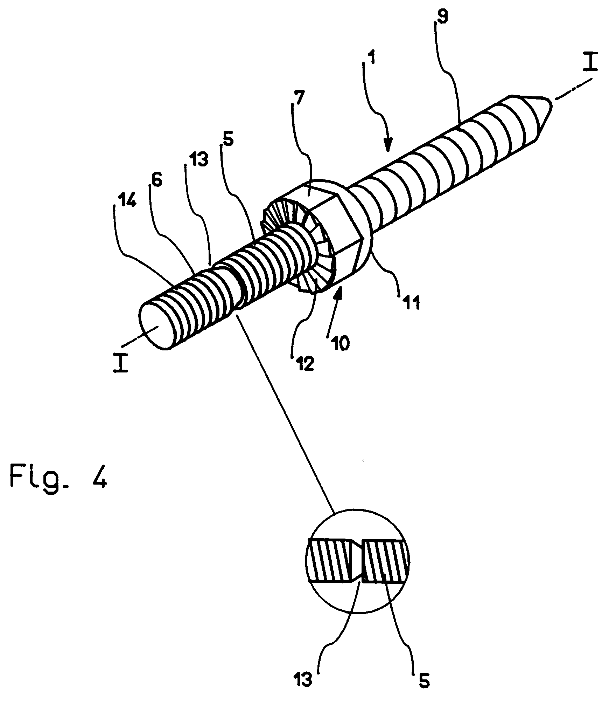

Dans le mode de réalisation de la figure 4, l'élément d'ancrage est une vis osseuse 1 à double filetage, la partie cylindrique filetée 5 à filetage externe 6 constituant le premier filetage, une seconde partie cylindrique coaxiale filetée 9 constituant un second filetage adapté pour se visser dans l'os, le plateau d'arrêt 7 constituant une partie intermédiaire de plus grande section séparant les deux parties filetées 5 et 9. Le plateau d'arrêt 7 comprend avantageusement une surface périphérique 10 à facettes en six pans, une surface de butée 11 se raccordant à la seconde partie cylindrique filetée 9 et destinée à venir porter contre l'os de vertèbre, et une face d'appui 12 généralement perpendiculaire à l'axe I-I de la partie cylindrique filetée 5 et se développant à partir de sa base.

La face d'appui 12 du plateau d'arrêt 7 comprend des premiers reliefs antiglissement. Par exemple, ces premiers reliefs antiglissement peuvent être une surface granuleuse, ou, de préférence, des nervures radiales réparties sur la face d'appui à partir de la base de la partie cylindrique filetée 5. La partie cylindrique filetée 5 comprend au moins une gorge annulaire intermédiaire 13, constituant une amorce de rupture pour faciliter le sectionnement de la partie excédentaire 14 après vissage d'un écrou sur la partie cylindrique filetée 5. La gorge 13 présente avantageusement une section en dent de scie, le fond de gorge étant adjacent à la partie restante de partie cylindrique filetée 5.

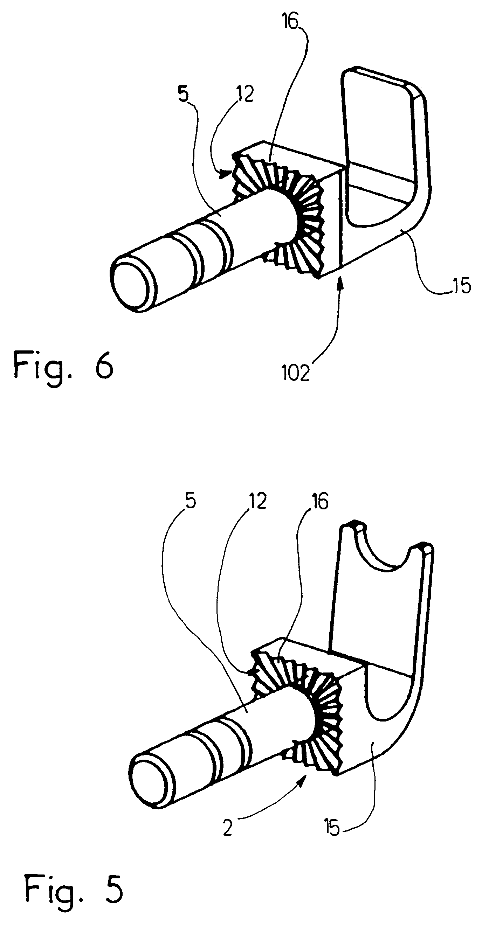

Dans le mode de réalisation de la figure 5, l'élément d'ancrage est un crochet pédiculaire 2, destiné à accrocher le pédicule d'une vertèbre. Le crochet 2 comporte un corps de crochet 15, recourbé comme le représente la figure, et une paroi arrière ou face d'appui 12 sur laquelle se raccorde la partie cylindrique filetée 5. La face d'appui 12 est généralement perpendiculaire à l'axe de la partie cylindrique filetée 5, et sert de plateau d'arrêt. Comme dans le cas de la vis pédiculaire de la figure 4, la face d'appui 12 comprend des premiers reliefs antiglissement tels que des nervures radiales 16.

Le mode de réalisation de la figure 6 représente un élément d'ancrage constitué par un crochet lamaire 102 conformé pour accrocher une lame de vertèbre. Le corps de crochet 15 est légèrement différent de celui de la figure 5, les autres éléments étant identiques, en particulier la face d'appui 12 avec les nervures radiales 16 autour de la base de la partie cylindrique filetée 5.

Dans l'un et l'autre des modes de réalisation des figures 5 et 6, les faces d'appui 12 du corps de crochet 15 constituent le plateau d'arrêt d'un tel élément d'ancrage, pour limiter le serrage d'un écrou sur la partie cylindrique filetée 5.

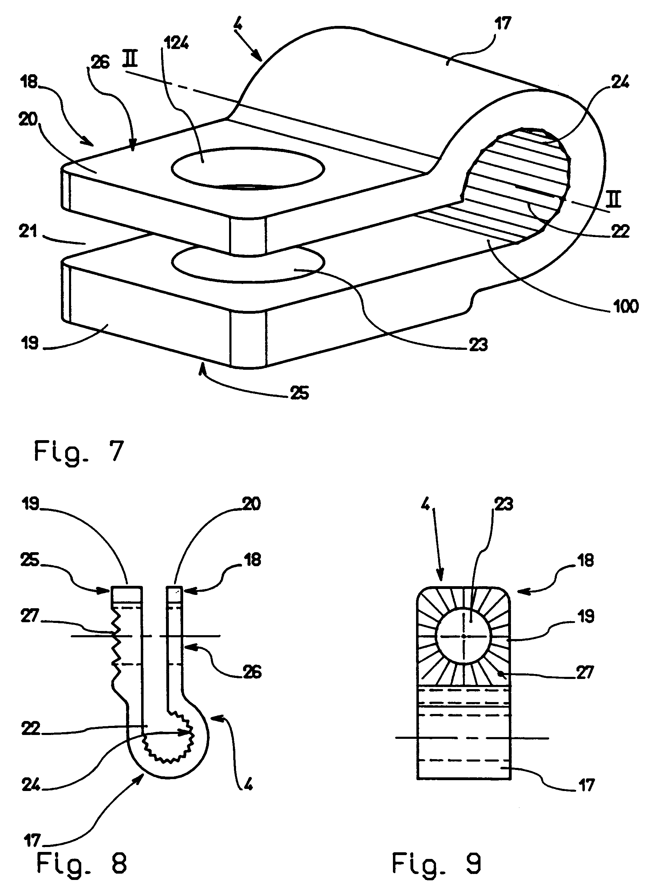

Dans le mode de réalisation des figures 7 à 9, le coulisseau de liaison 4 selon l'invention comprend une partie de serrage 17 et une partie de liaison 18.

La partie de liaison 18 comprend une première branche 19 et une seconde branche 20, généralement plates et séparées l'une de l'autre à l'état de repos par un intervalle 21, et se développant latéralement et parallèlement l'une à l'autre à partir de deux lèvres d'une fente longitudinale 100 de la partie de serrage 17 généralement cylindrique qui les relie l'une à l'autre. La partie de serrage 17 comporte une surface intérieure 22 généralement cylindrique, définissant un canal conformé pour entourer un tronçon de la tige de solidarisation 3. La partie de serrage 17 est déformable pour sélectivement être serrée ou desserrée sur la tige de solidarisation 3.

Chacune des branches 19 et 20 comprend un trou de passage respectivement 23 et 124, coaxiaux l'un à l'autre et d'axe commun perpendiculaire à l'axe de la surface intérieure 22 de partie de serrage 17. Les trous de passage 23 et 124 sont conformés pour recevoir la partie cylindrique filetée 5 d'un élément d'ancrage. On comprendra que le trou 124 pourrait, de façon sensiblement avantageuse, être remplacé par une fente autorisant l'inclinaison relative du coulisseau lors de son adaptation sur un élément d'ancrage.

Lorsqu'on introduit la partie cylindrique filetée 5 d'un élément d'ancrage 1, 2, 102 ou 202 dans les trous 23 et 124 d'un coulisseau 4 et que l'on serre l'écrou 8 à force jusqu'à écrasement des deux branches 19 et 20 l'une vers l'autre, il se produit une déformation de la partie de serrage 17 et une réduction du diamètre de la surface intérieure cylindrique 22 qui se serre sur la tige 3. L'élément d'ancrage, dans ce mode de réalisation, remplit ainsi la fonction simultanée d'élément de serrage.

La surface intérieure 22 de partie de serrage 17 comporte des seconds reliefs antiglissement, s'opposant à la rotation du coulisseau de liaison 4 autour de la tige de solidarisation 3 après serrage de l'élément de serrage. Par exemple, les seconds reliefs antiglissement peuvent avantageusement être des nervures longitudinales 24, de préférence à section transversale triangulaire, réalisant une surface de contact discontinue sur la tige de solidarisation 3.

Lors de l'utilisation, la première surface de contact 25 de la première branche 19 de partie de liaison 18 est destinée à venir porter contre la face d'appui 12 d'un élément d'ancrage. Cette première surface de contact 25 comprend également des premiers reliefs antiglissement correspondants, similaires de ceux prévus sur la face d'appui 12 de l'élément d'ancrage, pour s'opposer à toute rotation et à tout déplacement latéral du coulisseau de liaison 4 par rapport à l'élément d'ancrage après serrage de l'écrou 8 contre la surface de contact opposée 26 de la seconde branche 20 de partie de liaison 18.

Par exemple, les premiers reliefs antiglissement de la surface 25 sont des nervures radiales 27 telles que représentées sur les figures 8 et 9, entourant le trou 23, qui peuvent avantageusement avoir une forme complémentaire de celle des nervures radiales des éléments d'ancrage. Le coulisseau peut ainsi être verrouillé dans différentes positions angulaires sur l'élément d'ancrage. Et ce verrouillage évite le desserrage intempestif ultérieur des écrous 8.

On remarquera que, dans le mode de réalisation représenté, la première branche de liaison 19, comportant la première surface de contact 25, est décalée vers l'extérieur par rapport à l'axe II-II de la partie de serrage 17, ou axe de la surface cylindrique intérieure 22. De cette façon, la tige de solidarisation 3 se trouve légèrement décalée à l'écart de la vertèbre.

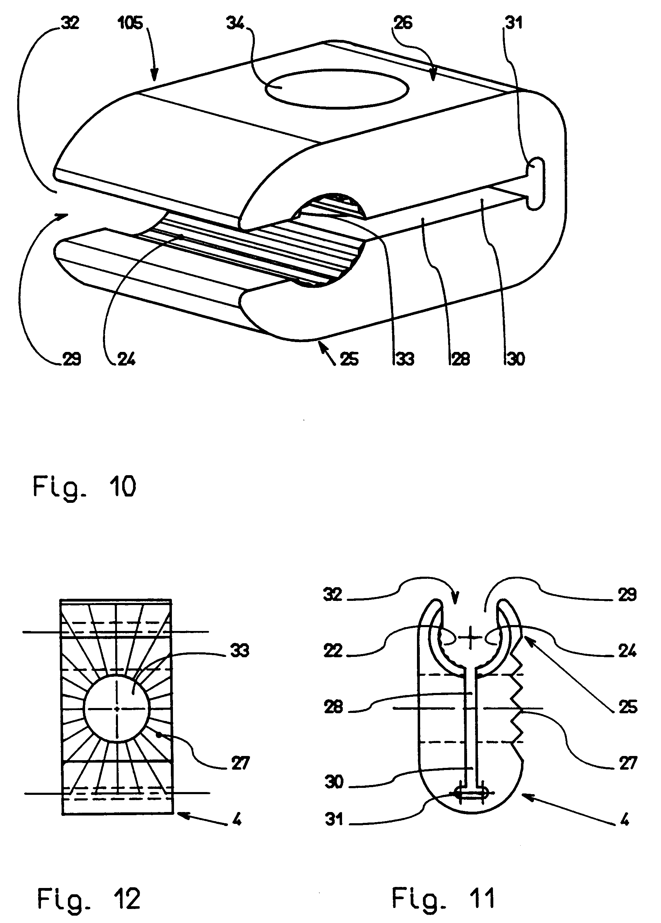

Dans le mode de réalisation des figures 10 à 12, le coulisseau de liaison 105 est de type ouvert, présentant une forme générale parallélépipédique rectangle, et est fendu axialement par une fente 28 comprenant une première partie 29 à section circulaire pour le passage de la tige de solidarisation 3, et une seconde partie 30 à section réduite terminée par un élargissement 31. L'ouverture 32 de fente permet l'introduction latérale de la tige de solidarisation 3 lorsque l'élément de serrage est desserré.

Comme dans le mode de réalisation précédent, le coulisseau 105 comprend un premier trou de passage 33 et un second trou de passage 34, se correspondant l'un à l'autre et conformés pour l'adaptation sur la partie cylindrique filetée 5 d'un élément d'ancrage.

La première surface de contact 25 est munie de premiers reliefs antiglissement tels que des nervures radiales 27. La surface intérieure 22 de la première partie 29 de fente est pourvue de seconds reliefs antiglissement tels que des nervures longitudinales 24, pouvant avantageusement présenter une section transversale triangulaire.

Ce coulisseau ouvert de la figure 10 permet l'insertion du coulisseau sur une partie intermédiaire de tige de solidarisation 3, sans avoir à introduire le coulisseau par une des extrémités de cette tige.

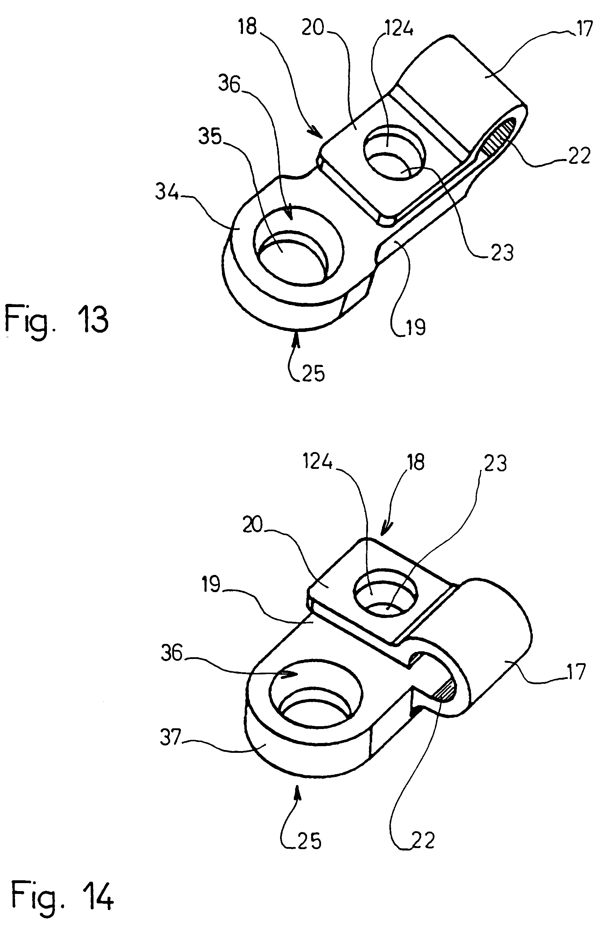

Dans le mode de réalisation de la figure 13, le coulisseau est similaire de celui des figures 7 à 9, avec une partie de serrage 17, une partie de liaison 18, une première branche 19 et une seconde branche 20 munies chacune d'un trou respectif 23 et 124.

Toutefois, ce mode de réalisation diffère en ce que le trou 23 de première branche 19 est taraudé, pour recevoir la tige filetée d'une vis dont la tête vient porter sur les bords du trou 124 lisse de passage et d'encastrement de la seconde branche 20, la vis assurant le serrage du coulisseau et son blocage sur la tige de solidarisation 3. Une seconde différence est que la première branche 19 comprend un prolongement latéral 34 lui-même muni d'un trou 35 pour le passage d'une tige d'élément d'ancrage. Le trou 35 peut avantageusement avoir une surface supérieure hémisphérique 36 destinée à recevoir la tête hémisphérique d'une vis osseuse conventionnelle. La surface inférieure du trou 35 peut elle-même être évasée, par exemple de forme conique tournée à l'opposé de la surface hémisphérique supérieure 36, de façon à permettre l'inclinaison de la tige de vis osseuse par rapport à l'axe du trou 35. La vis osseuse peut ainsi prendre diverses inclinaisons, indépendantes de la direction des axes du trou 35 et de la surface cylindrique 22.

On peut, dans ce cas, implanter une vis osseuse dans une vertèbre, après l'avoir introduite dans le trou 35 du prolongement latéral 34, et visser ensuite une vis dans les trous 23 et 124 jusqu'à rapprochement des branches 19 et 20 et serrage de la partie de serrage 17 sur la tige de solidarisation 3.

La surface cylindrique 22 comprend des nervures longitudinales, comme dans les modes de réalisation précédents. Il en est de même de la première surface de contact 25, ou surface inférieure du prolongement latéral 34, qui comporte des reliefs antiglissement, tels que des nervures radiales. On comprendra qu'un tel coulisseau de la figure 13 peut être utilisé à la place des coulisseaux des figures 7 à 12, en présentant les mêmes effets de blocage antirotation.

La figure 14 représente un coulisseau selon un autre de mode de réalisation similaire de celui de la figure 13, et dans lequel les éléments similaires ont été repérés par les mêmes références numériques. La différence est que le prolongement 37 de la première branche 19 se développe longitudinalement et non transversalement, c'est-à-dire dans le sens de l'axe longitudinal de la surface cylindrique 22.

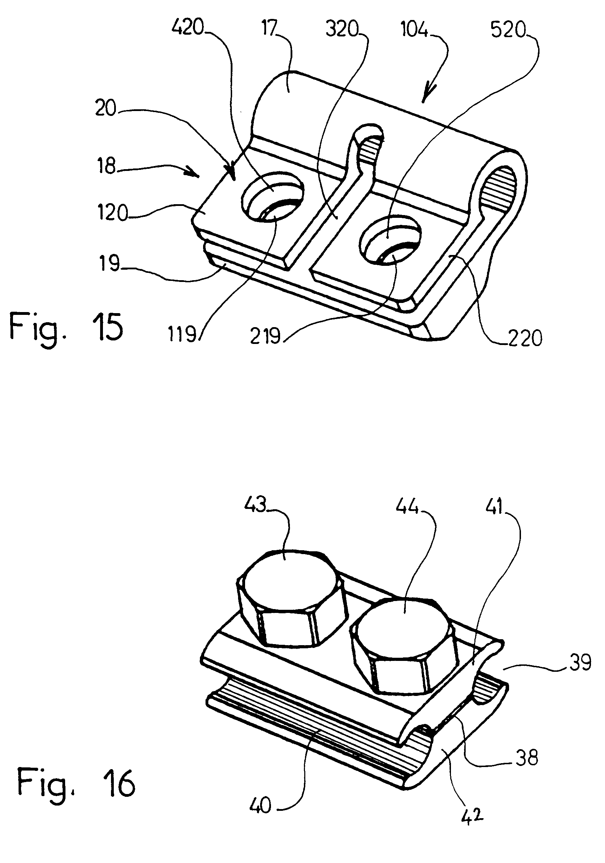

La figure 15 représente un coulisseau de liaison double 104, dans lequel la première branche 19 de la partie de liaison 18 est en une partie, tandis que la seconde branche 20 de la partie de liaison 18 est en deux parties respectivement 120 et 220, séparées l'une de l'autre par une échancrure transversale 320 et munies chacune de trous de passage 420 et 520 pour le passage de deux vis de serrage se vissant dans des trous taraudés correspondants 119 et 219 de la première branche 19. De préférence, le coulisseau 104 de la figure 15 a une longueur supérieure à celle du coulisseau des modes de réalisation précédents, par exemple une longueur double. Une telle variante permet de joindre bout à bout deux tiges de solidarisation successives pour le traitement du rachis d'un patient.

La figure 16 représente un autre mode de réalisation de coulisseau double, qui présente l'avantage d'être ouvert, comportant une fente traversante 38 terminée par deux parties cylindriques ouvertes 39 et 40 recevant chacune une tige de solidarisation. Le coulisseau est ainsi formé de deux pièces 41 et 42 serrées l'une contre l'autre par deux vis 43 et 44, pour le serrage sur deux tiges de solidarisation parallèles. Pour éviter la rotation des tiges de solidarisation, les surfaces cylindriques 39 et 40 sont pourvues de nervures longitudinales, comme représenté sur la figure.

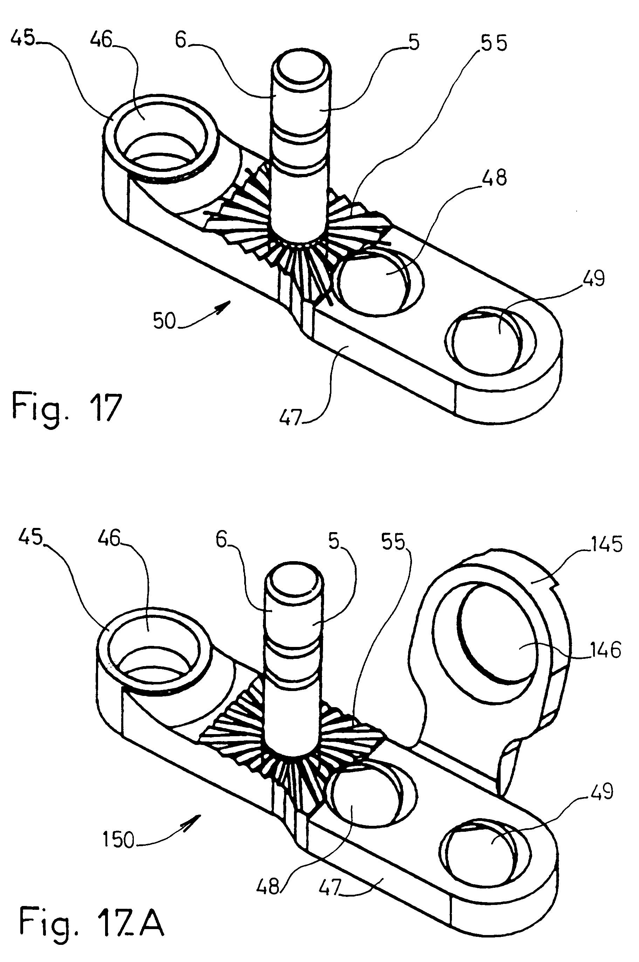

La figure 17 représente un autre type d'élément d'ancrage selon l'invention, à savoir une plaque d'appui sacré 50. Une telle plaque peut être utilisée pour constituer un appui intermédiaire entre les vertèbres et un coulisseau selon les modes de réalisation des figures 7 à 12, 13 ou 14.

La plaque d'appui sacré 50 se présente sous la forme d'une plaque allongée munie, dans sa partie médiane, d'une partie cylindrique filetée 5 à filetage extérieur 6. A l'une de ses extrémités 45, d'épaisseur renforcée, la plaque d'appui sacré comprend un alésage 46 conformé pour recevoir une tête de vis osseuse standard, destinée à être implantée dans le pédicule de la vertèbre S1. A son autre extrémité 47, la plaque d'appui sacré comprend deux autres alésages 48 et 49 servant à la réception de têtes de vis osseuses standard implantées dans le sacrum.

La face supérieure de la plaque d'appui sacré 50 comprend, autour de la base de la partie cylindrique filetée 5, des premiers reliefs antiglissement tels que des nervures radiales 55, pour coopérer avec les premiers reliefs antiglissement correspondants des coulisseaux.

L'axe de l'alésage 46 est sensiblement perpendiculaire au plan des faces inférieure et supérieure de la plaque d'appui sacré 50, et il est sensiblement parallèle à l'axe de la partie cylindrique filetée 5. Par contre, les axes respectifs des alésages 48 et 49 sont obliques, pour orienter les vis osseuses qu'ils contiennent vers l'extérieur et faciliter ainsi la fixation de la plaque d'appui sacré sur le sacrum dans l'aileron sacré.

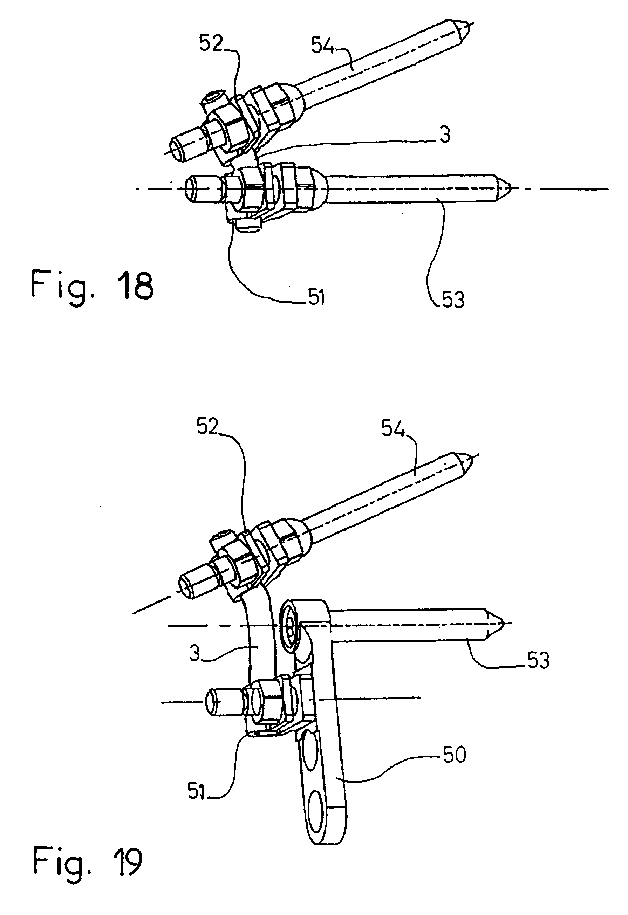

La plaque d'appui sacré 50 permet d'augmenter l'espacement entre deux coulisseaux successifs 51 et 52 sur la tige de solidarisation 3, le premier coulisseau 51 assurant la solidarisation à une première vis osseuse 53 implantée en S1 par l'intermédiaire de la plaque d'appui sacré 50, le second coulisseau 52 assurant la liaison avec une seconde vis osseuse 54 implantée en L5. Ainsi, la figure 18 représente un arrangement traditionnel dans lequel deux vis osseuses 53 et 54 sont reliées l'une à l'autre seulement par les coulisseaux 51 et 52, qui sont très rapprochés l'un de l'autre sur la tige de solidarisation 3. La proximité rend difficile le positionnement des vis et des coulisseaux. Par contre, sur la figure 19, on constate que l'interposition de la plaque d'appui sacré 50 entre la vis osseuse 53 et le coulisseau 51 augmente la distance entre les coulisseaux 51 et 52, facilitant considérablement le positionnement des éléments.

On choisit avantageusement les dimensions de la plaque d'appui sacré 50 de façon que l'augmentation d'écartement des coulisseaux 51 et 52 soit d'environ 15 millimètres.

La figure 17A illustre une plaque d'appui illio-sacré 150, qui reprend les éléments de la plaque d'appui sacré 50 de la figure 17, repérés par les mêmes références numériques, en ajoutant une patte latérale d'ancrage 145 recourbée à angle droit comme représenté et munie d'un alésage latéral 146 conformé pour recevoir une tête de vis osseuse standard.

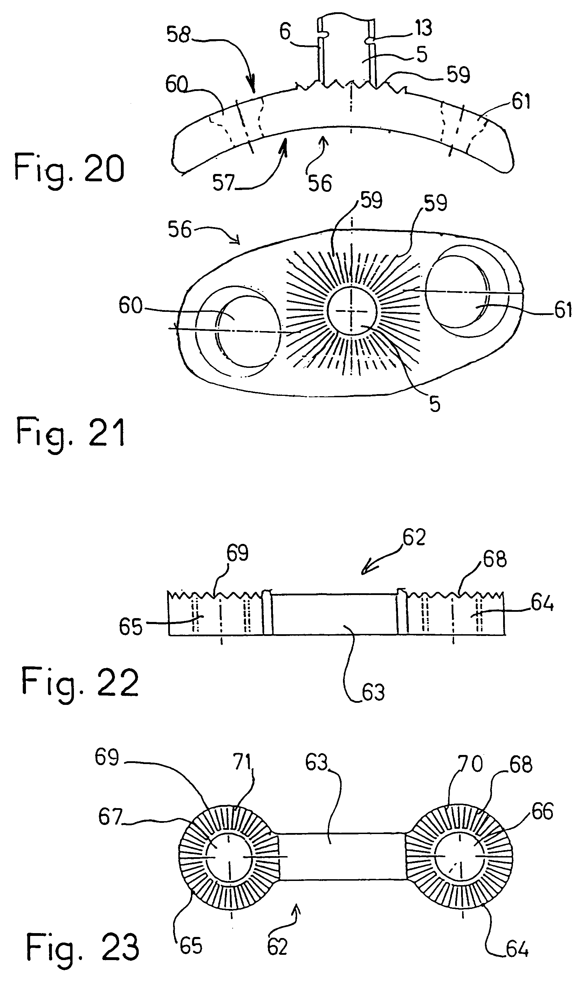

Les figures 20 et 21 illustrent un autre type d'élément d'ancrage selon l'invention, sous forme d'une plaque d'appui 56 sur le corps d'une vertèbre. Cette plaque d'appui 56 se présente sous forme d'une plaque rigide, généralement ovale, dont les faces opposées inférieure 57 et supérieure 58 sont incurvées pour épouser la forme du corps vertébral. La face supérieure 58 convexe comporte sur sa partie médiane des reliefs antiglissement 59 et une partie cylindrique filetée 5 pour adaptation d'un coulisseau. La face supérieure 58 avec ses reliefs antiglissement 59 constitue un plateau d'arrêt.

La plaque d'appui 56 est munie de deux alésages 60 et 61, situés de part et d'autre de la partie cylindrique filetée 5, et qui sont tous deux de forme généralement hémisphérique, pour recevoir la tête hémisphérique complémentaire d'une vis osseuse standard. On remarquera que les axes des alésages 60 et 61 sont décalés, comme représenté sur la figure 21, de façon que les vis osseuses pénètrent dans la vertèbre sans se toucher, malgré l'inclinaison de leurs axes respectifs.

Etant donné que l'appareil selon l'invention peut comporter deux tiges de solidarisation 3 situées de part et d'autre de l'apophyse épineuse des vertèbres, pour mieux soutenir et corriger le rachis d'un patient, il peut être avantageux de solidariser ces deux tiges 3 par un ou plusieurs systèmes de stabilisation transversale, tel que représenté sur les figures 22 et 23.

Ce système de stabilisation comprend une entretoise 62, comportant une partie centrale 63 se terminant par deux parties d'extrémité évasées 64 et 65 munies chacune d'un trou de passage respectif 66 et 67 et d'une face d'appui 68 et 69 munie de premiers reliefs antiglissement tels que des nervures radiales 70 et 71. De préférence, les trous 66 et 67 sont taraudés. La liaison de l'entretoise 62 avec les tiges de solidarisation 3 s'effectue par l'intermédiaire de coulisseaux de liaison, dont les premières surfaces de contact 25 viennent en appui sur les faces d'appui nervurées 68 et 69 de l'entretoise, interdisant toute rotation. Des vis assurent le serrage de chaque coulisseau sur la face correspondante 68 ou 69 de l'entretoise 62. De cette façon, on peut régler l'écartement des deux tiges de solidarisation 3 en décalant longitudinalement les coulisseaux l'un par rapport à l'autre, l'entretoise 62 se trouvant alors en position oblique.

L'appareil de l'invention permet l'utilisation d'une tige ou de plusieurs éléments de tige, de courbure appropriée à la région vertébrale à traiter.

La mise en place des coulisseaux 4 sur la tige de solidarisation 3 s'effectue aisément, puisqu'ils sont enfilés par chacune des extrémités de cette dernière, ou par engagement latéral, et ils coulissent librement sur la tige 3 quelle que soit sa courbure.

Le principe de réduction du rachis déformé est tridimensionnel. Il faut transformer une courbe scoliotique orientée dans un plan proche du plan frontal en une courbe d'allure physiologique située dans le plan sagittal et présentant une courbure cyphotique, thoracique et lordotique lombaire normale.

On donne tout d'abord à la tige de solidarisation une forme proche de la courbure physiologique normale, et on la positionne dans le plan sagittal du patient, en fixant ses deux extrémités par des éléments d'ancrage et des coulisseaux correctement serrés. Les reliefs antiglissement de l'invention permettent ainsi un blocage très efficace de la tige, à la fois en translation et surtout en rotation. On positionne des éléments d'ancrage dans ou sur les autres vertèbres intermédiaires. Les ancrages les plus proches de la tige sont engagés dans les coulisseaux et verrouillés pour bloquer la tige dans le plan sagittal et éviter également sa rotation. L'engagement des ancrages les plus éloignés situés dans la concavité de la courbe est permis par la longueur de la partie cylindrique filetée 5 des éléments d'ancrage. Une fois l'extrémité de la partie cylindrique filetée passée dans l'alésage du coulisseau 4 de liaison lui-même adapté sur la tige mais non serré, le serrage progressif de l'écrou 8 rapproche le point d'ancrage de la tige de solidarisation, réalisant la réduction progressive de courbure vertébrale. Si l'élasticité du rachis s'épuise, la tige de solidarisation 3 se déforme, et assure une correction dans une position intermédiaire de réduction maximale.

L'appareil selon l'invention permet ainsi d'aller rechercher sur place chaque élément d' ancrage intermédiaire, pour introduire sa partie cylindrique filetée dans l'alésage d'un coulisseau, et de le fixer rigidement sur la tige, en alignement avec les éléments d'ancrage supérieur et inférieur, pour obtenir le redressement voulu des vertèbres ainsi appareillées par vissage et serrage des écrous provoquant un blocage des coulisseaux sur la tige.

La mise en place de l'appareil faisant l'objet de l'invention sur le rachis du patient peut s'effectuer aussi bien en position antérieure qu'en position postérieure, par rapport au rachis.

Grâce à la présence des reliefs antiglissement, la mise en place de l'appareil selon l'invention peut s'effectuer dans un temps opératoire nettement plus court qu'avec les appareils connus antérieurement, étant donné la facilité accrue avec laquelle le chirurgien peut serrer ou desserrer les écrous 8 de serrage de coulisseau sans modifier la pénétration des vis osseuses dans l'os.

On comprendra qu'un appareil selon l'invention peut comprendre, selon le traitement de rachis à réaliser, des ancrages à vis, ou des ancrages à crochets, ou des ancrages à plaques, ou une combinaison de deux ou trois de ces types d'ancrage qui sont ainsi indépendants les uns des autres.

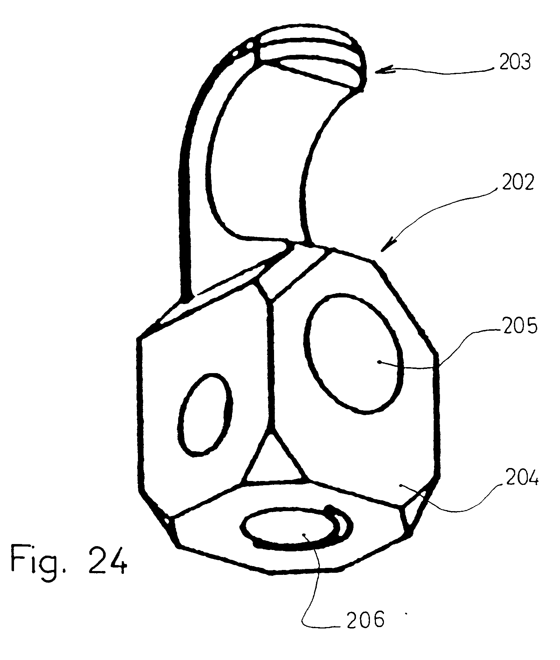

En outre, les éléments particuliers de l'invention permettent un assemblage de deux crochets en opposition, associés à des coulisseaux respectifs montés sur une même tige de solidarisation 3, pour former une pince pédiculo-lamaire. Un tel assemblage est illustré par les crochets 200 et 202 en partie supérieure de l'appareil des figures 1 à 3. Le crochet pédiculaire 200 est conforme au type de crochet 2 représenté sur la figure 5. Le crochet 202 est un crochet lamaire d'extrémité représenté sur la figure 24, dans lequel la partie recourbée en crochet 203 est solidaire d'un corps 204 généralement cubique percé d'un alésage transversal 205 de passage de tige de solidarisation 3. Une vis adaptée dans un trou taraudé axial 206 vient en appui sur la tige traversant l'alésage transversal 205 et bloque le crochet 202 sur la tige. La partie de crochet 203 se trouve proche de l'axe de la tige de solidarisation 3. Les deux crochets 200 et 202 en opposition peuvent être adaptés l'un pour être en appui pédiculaire et l'autre en appui lamaire sur une même vertèbre, en respectant le décalage naturel entre la lame et le pédicule. Grâce au verrouillage en rotation des crochets pédiculaires, obtenu par les reliefs antiglissement, on réalise un accrochage particulièrement efficace sur certaines vertèbres supérieures sans avoir recours à des éléments d'ancrage à vis.

On comprendra qu'une telle pince pédiculo-lamaire peut être utilisée indépendamment de la présence ou de la nature d'autres éléments d'ancrage, et qu'elle présente ses fonctionnalités et ses avantages propres.

La présente invention n'est pas limitée aux modes de réalisation qui ont été explicitement décrits, mais elle en inclut les diverses variantes et généralisations contenues dans le domaine des revendications ci-après.

## Revendications

1. Appareil de traitement du rachis, comprenant :
- des éléments d'ancrage (1, 2, 200, 202, 50, 56) dans les vertèbres, comportant une partie d'ancrage (9, 15, 50, 150, 56) conformée pour s'ancrer dans ou sur l'os d'une vertèbre et prolongée par une partie cylindrique filetée (5) sur laquelle se visse un écrou de serrage (8),
- au moins une tige de solidarisation (3), à section circulaire et à surface extérieure lisse,
- des coulisseaux de liaison (4) pour relier des éléments d'ancrage (1, 2, 200, 202, 50, 56) à la tige de solidarisation (3), les coulisseaux de liaison (4) comportant une partie de serrage (17) et une partie de liaison (18), la partie de serrage (17) présentant des surfaces intérieures (22) conformées pour entourer un tronçon de la tige de solidarisation (3) et étant déformable pour sélectivement être serrée ou desserrée sur la tige de solidarisation (3), la partie de liaison (18) comportant une première (19) et une seconde (20) branches se développant latéralement et parallèlement l'une à l'autre à partir de deux lèvres d'une fente longitudinale (100) de la partie de serrage (17), lesdites branches (19, 20) de partie de liaison comportant des trous de passage correspondants (23, 124) pour recevoir la partie cylindrique filetée (5) d'un élément d'ancrage permettant de serrer les branches (19, 20) l'une vers l'autre et de serrer ainsi la partie de serrage (17),
caractérisé en ce que :
- les éléments d'ancrage (1, 2, 200 202, 50, 56) comprennent un plateau d'arrêt (7) à face d'appui (12) généralement perpendiculaire à l'axe de partie cylindrique filetée (5) et se développant à partir de sa base,
- la face d'appui (12) du plateau d'arrêt (7) comprend des premiers reliefs antiglissement (16),
- la première surface de contact (25) correspondante de première branche (19) de partie de liaison (18), destinée à venir porter contre la face d'appui (12) d'élément d'ancrage, comprend des premiers reliefs antiglissement (27) correspondants pour s'opposer à toute rotation et à tout déplacement latéral du coulisseau de liaison (4) par rapport à l'élément d'ancrage après serrage de l'écrou (8) contre la seconde surface de contact opposée (26) de seconde branche (20) de partie de liaison (18).

2. Appareil selon la revendication 1, caractérisé en ce que les premiers reliefs antiglissement (16, 27) comprennent des nervures radiales réparties respectivement sur la face d'appui (12) à partir de la base de la partie cylindrique filetée (5) et sur la première surface de contact (25) à partir du bord du trou de passage correspondant (23).

3. Appareil selon l'une des revendications 1 ou 2, caractérisé en ce que les surfaces intérieures (22) de partie de serrage (17) comportent des seconds reliefs antiglissement (24), s'opposant à la rotation du coulisseau de liaison (4) autour de la tige de solidarisation (3) après serrage de l'élément de serrage.

4. Appareil selon la revendication 3, caractérisé en ce que les seconds reliefs antiglissement (24) comprennent des nervures longitudinales.

5. Appareil selon l'une quelconque des revendications 1 à 4, caractérisé en ce qu'il comprend, comme élément d'ancrage, une ou plusieurs vis osseuses à double filetage (1), la partie cylindrique filetée (5) d'élément d'ancrage constituant le premier filetage, une seconde partie cylindrique coaxiale filetée (9), opposée au plateau d'arrêt (7), constituant la partie d'ancrage à second filetage adapté pour se visser dans l'os.

6. Appareil selon l'une quelconque des revendications 1 à 5, caractérisé en ce qu'il comprend, comme élément d'ancrage, un ou plusieurs crochets (2) dont le corps de crochet (15) constitue la partie d'ancrage et dans lesquels la paroi arrière des crochets, perpendiculaire à l'axe de la partie cylindrique filetée (5), sert de plateau d'arrêt et comporte les premiers reliefs antiglissement (16).

7. Appareil selon l'une quelconque des revendications 1 à 6, caractérisé en ce qu'il comprend, comme élément d'ancrage, une plaque d'appui sacré (50) et/ou une plaque d'appui illio-sacré (150), de forme générale allongée, comportant dans sa partie médiane la partie cylindrique filetée (5) bordée de la surface d'appui à premier relief antiglissement (55), comportant au voisinage d'une première extrémité (45) une partie d'épaisseur renforcée percée d'un alésage (46) de passage de vis osseuse standard, et comportant au voisinage de sa seconde extrémité (47) deux autres alésages (48, 49) de passage de vis osseuse standard à axe incliné.

8. Appareil selon l'une quelconque des revendications 1 à 7, caractérisé en ce qu'il comprend, comme élément d'ancrage, une plaque d'appui (56) sur le corps d'une vertèbre, de forme ovale et incurvée, comportant la partie cylindrique filetée (5) sur la partie médiane de sa face convexe (58) à relief antiglissement (59), et, de part et d'autre de cette partie médiane, deux alésages (60, 61) de passage de vis osseuses à axes décalés.

9. Appareil selon l'une quelconque des revendications 1 à 8, caractérisé en ce qu'il comprend un coulisseau de liaison double (104), dans lequel l'une au moins (20) des branches (19, 20) de la partie de liaison (18) comporte deux parties (120, 220) séparées par une échancrure transversale (320) et munies chacune de trous (420, 520) de passage de vis de serrage correspondant à des trous taraudés (119, 219) ménagés sur l'autre branche (19).

10. Appareil selon l'une quelconque des revendications 1 à 9, caractérisé en ce qu'il comprend au moins un coulisseau de liaison (4) tel que :
- le coulisseau de liaison (4) comprend deux branches (19, 20) généralement plates séparées l'une de l'autre et se raccordant chacune à une partie de serrage (17) généralement cylindrique qui les relie l'une à l'autre,
- la première branche de liaison (19), comportant la première surface de contact (25) destinée à porter contre la face d'appui (12) d'élément d'ancrage, est décalée vers l'extérieur par rapport à l'axe (II-II) de la partie de serrage (17).

11. Appareil selon la revendication 10, caractérisé en ce que :
- les branches de liaison (19, 20) du coulisseau de liaison (4) comportent chacune un seul trou de passage (23, 124),
- l'élément d'ancrage est une vis osseuse (1) à double filetage selon la revendication 5, qui remplit simultanément la fonction d'élément de serrage et d'élément d'ancrage.

12. Appareil selon l'une quelconque des revendications 1 à 9, caractérisé en ce que le coulisseau de liaison (105) est généralement de forme parallélépipédique, fendu axialement par une fente (28) comportant une première partie (29) à section circulaire pour le passage de la tige de solidarisation (3) et une seconde partie (30) à section réduite terminée par un élargissement (31), l'ouverture (32) de fente permettant l'introduction latérale de la tige de solidarisation (3) lorsque l'élément de serrage est desserré.

13. Appareil selon la revendication 10, caractérisé en ce que :
- la première branche de liaison (19), comportant la première surface de contact (25), comprend un prolongement latéral (34) ou axial (37) non recouvert par la seconde branche de liaison (20) et muni d'un trou (35) de passage de vis pédiculaire,
- la seconde branche de liaison (20) comporte un trou (124) lisse de passage et d'encastrement pour une tête de vis de serrage dont la tige se visse dans un trou taraudé (23) correspondant de la première branche de liaison (19).

14. Appareil selon l'une quelconque des revendications 1 à 13, caractérisé en ce qu'il comprend un assemblage de deux crochets (200, 202) en opposition associés à des coulisseaux respectifs (4) montés sur une même tige de solidarisation (3), adaptés l'un pour être en appui lamaire et l'autre pour être en appui pédiculaire sur une même vertèbre.

15. Appareil selon l'une quelconque des revendications 1 à 14, caractérisé en ce qu'il comprend :
- deux tiges de solidarisation (3) avec leurs éléments d'ancrage (1) et leurs coulisseaux (4) respectifs,
- au moins une entretoise (62) reliée à chaque tige (3) de solidarisation par des coulisseaux respectifs (4) contre lesquels elle porte par des surfaces (68, 69) à premiers reliefs antiglissement (70, 71).

## Claims

1. Spinal therapy device comprising:
- vertebral anchoring elements (1, 2, 200, 202, 50, 56) having an anchoring part (9, 15, 50, 150, 56) shaped to be anchored in or on the bone of a vertebra and extended by a screwthreaded cylindrical part (5) onto which a clamping nut (8) is screwed,
- at least one circular cross-section fastening rod (3) having a smooth outside surface,
- connecting slides (4) for connecting anchoring elements (1, 2, 200, 202, 50, 56) to the fastening rod (3), the connecting slides (4) having a clamping part (17) and a connecting part (18), the clamping part (17) having inside surfaces (22) shaped to surround a section of the fastening rod (3) and being deformable for selectively clamping it to and releasing it from the fastening rod (3), the connecting part (18) having a first branch (19) and a second branch (20) extending laterally and parallel to each other from two lips of a longitudinal slot (100) in the clamping part (17), said branches (19, 20) of the connecting part having corresponding through-holes (23, 124) for receiving the screwthreaded cylindrical part (5) of an anchoring element for clamping the branches (19, 20) together and thereby clamping the clamping part (17),
characterised in that:
- the anchoring elements (1, 2, 200, 202, 50, 56) include a locking plate (7) with a bearing surface (12) generally perpendicular to the axis of the screwthreaded cylindrical part (5) and extending from its base,
- the bearing surface (12) of the locking plate (7) includes first non-slip projections (16),
- the corresponding first contact surface (25) of the first branch (19) of the connecting part (18), adapted to bear against the bearing surface (12) of the anchoring element, includes corresponding first non-slip projections (27) to oppose any rotation and any lateral displacement of the connecting slide (4) relative to the anchoring element after clamping of the nut (8) against the opposite second contact surface (26) of the second branch (20) of the connecting part (18).

2. Device according to claim 1, characterised in that the first non-slip projections (16, 27) comprise radial ribs respectively disposed on the bearing surface (12) from the base of the screwthreaded cylindrical part (5) and on the first contact surface (25) from the edge of the corresponding through-hole (23).

3. Device according to claim 1 or claim 2, characterised in that the inside surfaces (22) of the clamping part (17) have second non-slip projections (24) opposing rotation of the connecting slide (4) about the fastening rod (3) after clamping of the clamping member.

4. Device according to claim 3, characterised in that the second non-slip projections (24) comprise longitudinal ribs.

5. Device according to any one of claims 1 to 4, characterised in that it includes, as anchoring element(s), one or more double-threaded bone screws (1), the screwthreaded cylindrical part (5) of an anchoring element constituting the first screwthread, a coaxial second cylindrical screwthreaded part (9) on the opposite side of the locking plate (7) constituting the anchoring part with a second screwthread adapted to be screwed into bone.

6. Device according to any one of claims 1 to 5, characterised in that it includes, as anchoring element(s), one or more hooks (2) in which the hook body (15) constitutes the anchoring part and in which the rear wall of the hook, perpendicular to the axis of the screwthreaded cylindrical part (5), provides a locking plate and carries the first non-slip projections (16).

7. Device according to any one of claims 1 to 6, characterised in that it includes, as anchoring element(s), a sacral bearing plate (50) and/or an illio-sacral bearing plate (150), of generally elongate shape, having in its middle part the screwthreaded cylindrical part (5) flanked by the bearing surface with first non-slip projections (55), having in the vicinity of a first end (45) a thicker part through which is a bore (46) for a standard bone screw, and having near its second end (47) two other bores (48, 49) for standard bone screws with the axis inclined.

8. Device according to any one of claims 1 to 7 characterised in that it comprises, as clamping member(s), a bearing plate (56) adapted to bear on the body of a vertebra, having a curved oval shape with the screwthreaded cylindrical part (5) on the middle part of its convex side (58) with non-slip projections (59), and, on either side of said middle part, two bores (60, 61) for bone screws with offset axes.

9. Device according to any one of claims 1 to 8, characterised in that it includes a double connecting slide (104), in which at least one branch (20) of the branches (19, 20) of the connecting part (18) has two parts (120, 220) separated by a transverse notch (320) and each having holes (420, 520) for clamping screws corresponding to screwthreaded holes (119, 219) on the other branch (19).

10. Device according to any one of claims 1 to 9 characterised in that it includes at least one connecting slide (4) such that:
- the connecting slide (4) has two generally flat branches (19, 20) separated from each other and each merging with a generally cylindrical clamping part (17) which links them together,
- the first connecting branch (19), including the first contact surface (25) adapted to bear against the bearing surface (12) of the anchoring element, is offset outwardly relative to the axis (II-II) of the clamping part (17).

11. Device according to claim 10, characterised in that:
- the connecting branches (19, 20) of the connecting slide (4) each include a single through-hole (23, 124),
- the anchoring element is a double-threaded bone screw (1), as in claim 5, which simultaneously serves as a clamping member and an anchoring element.

12. Device according to any one of claims 1 to 9, characterised in that the connecting slide (105) is of generally parallelepiped shape, slit axially by a slot (28) having a circular cross-section first part (29) for the fastening rod (3) to pass through and a smaller cross-section second part (30) ending at an enlargement (31), the slot opening (32) enabling lateral insertion of the fastening rod (3) when the clamping member is released.

13. Device according to claim 10, characterised in that:
- the first connecting branch (19), including the first contact surface (25), includes a lateral extension (34) or an axial extension (37) not covered by the second connecting branch (20) and having a hole (35) for a pedicle screw to pass through,
- the second connecting branch (20) includes a smooth through-hole (124) for receiving the head of a clamping screw whose shank is screwed into a corresponding screwthreaded hole (23) on the first connecting branch (19).

14. Device according to any one of claims 1 to 13, characterised in that it includes an assembly of two opposed hooks (200, 202) associated with respective slides (4) mounted on a common fastening rod (3), one adapted for bearing engagement on the lamina and the other for bearing engagement on the pedicle of the same vertebra.

15. Device according to any one of claims 1 to 14, characterised in that it includes:
- two fastening rods (3) with their respective anchoring elements (1) and slides (4),
- at least one spacer (62) connected to each fastening rod (3) by respective slides (4) on which it bears through surfaces (68, 69) with first non-slip projections (70, 71).

## Patentansprüche

1. Vorrichtung zur Behandlung der Wirbelsäule, bestehend aus:
- Verankerungselementen (1, 2, 200, 202, 50, 56) in den Wirbeln, die ein Verankerungsteil (9, 15, 50, 150, 56) aufweisen, das für ein Verankern in oder auf dem Knochen eines Wirbels angepaßt und mit einem zylindrischen Teil verlängert ist, das mit einem Außengewinde (5) versehen ist, auf welches eine Festklemmutter (8) aufschraubbar ist,
- wenigstens einem Versteifungsschaft (3) mit einem kreisförmigen Querschnitt und einer glatten Außenfläche,
- Verbindung-Gleitstücken (4) zum Verbinden der Verankerungselemente (1, 2, 200, 202, 50, 56) mit dem Versteifungsschaft (3), wobei die Verbindung-Gleitstücke (4) ein Festklemmteil (17) und ein Verbindungsteil (18) aufweisen, wobei das Festklemmteil (17) Innenflächen (22) darbietet, die für ein Umfassen eines Teilstückes des Versteifungsschaftes (3) angepaßt und verformbar sind, um an dem Versteifungsschaft (3) selektiv festgeklemmt oder davon losgelöst zu sein, wobei das Verbindungsteil (18) einen ersten (19) und einen zweiten (20) Schenkel aufweist, die sich seitlich von und parallel zueinander von zwei Lippen eines Längsschlitzes (100) des Festklemmteils (17) aus entfalten, wobei diese Schenkel (19, 20) des Verbindungsteils korrespondierende Durchgangslöcher (23, 124) aufweisen, um das zylindrische Gewindeteil (5) eines Verankerungselements aufzunehmen, welches sowohl die Schenkel (19, 20) gegeneinander festzuklemmen erlaubt wie auch ein Festklemmen des Festklemmteils (17),
dadurch gekennzeichnet, daß
- die Verankerungselemente (1, 2, 200, 202, 50, 56) ein Anschlagplateau (7) mit einer Abstützfläche (12) aufweisen, die zu der Achse des zylindrischen Gewindeteils (5) generell senkrecht verläuft und sich von ihrer Basis aus entfaltet,
- die Abstützfläche (12) des Anschlagplateaus (7) erste rutschfeste Erhebungen (16) aufweist,
- die erste korrespondierende Berührungsfläche (25) des ersten Schenkels (19) des Verbindungsteils (18), die für eine Anlage an der Abstützfläche (12) des Verankerungselements bestimmt ist, erste korrespondierende rutschfeste Erhebungen (27) aufweist, um jeder Drehung und jeder seitlichen Verschiebung des Verbindung-Gleitstückes (4) in Bezug auf das Verankerungselement nach dem Festklemmen der Mutter (8) an der zweiten gegenüberliegenden Berührungsfläche (26) des zweiten Schenkels (20) des Verbindungsteils (18) entgegenzuwirken.

2. Vorrichtung nach Anspruch 1, dadurch gekennzeichnet, daß die rutschfesten ersten Erhebungen (16, 27) radiale Rippen aufweisen, die auf der Abstützfläche (12) von der Basis des zylindrischen Gewindeteils (5) aus und auf der ersten Berührungsfläche (25) von dem Rand des korrespondierenden Durchgangsloches (23) aus voneinander beabstandet sind.

3. Vorrichtung nach einem der Ansprüche 1 oder 2, dadurch gekennzeichnet, daß die Innenflächen (22) des Festklemmteils (17) zweite rutschfeste Erhebungen (24) aufweisen, welche der Drehung des Verbindung-Gleitstückes (4) um den Versteifungsschaft (3) herum nach dem Festklemmen des Festklemmelements entgegenwirken.

4. Vorrichtung nach Anspruch 3, dadurch gekennzeichnet, daß die zweiten rutschfesten Erhebungen (24) Längsrippen aufweisen.

5. Vorrichtung nach einem der Ansprüche 1 bis 4, dadurch gekennzeichnet, daß sie als Verankerungselement eine oder mehrere Doppelgewinde-Knochenschrauben (1) aufweist, wobei der zylindrische Gewindeteil (5) des Verankerungselements das erste Gewinde bildet und ein zweiter koaxialer zylindrischer Gewindeteil (9) gegenüberliegend zu dem Anschlagplateau (7) den Verankerungsteil eines zweiten Gewindes bildet, das für ein Einschrauben in den Knochen angepaßt ist.

6. Vorrichtung nach einem der Ansprüch 1 bis 5, dadurch gekennzeichnet, daß sie als Verankerungselement einen oder mehrere Haken (2) aufweist, bei welchen der Hakenkörper (15) den Verankerungsteil bildet und in welchen die hintere Wand der Haken senkrecht zu der Achse des zylindrischen Gewindeteils (5) als Anschlagplateau dient und die ersten rutschfesten Erhebungen (16) aufweist.

7. Vorrichtung nach einem der Ansprüche 1 bis 6, dadurch gekennzeichnet, daß sie als Verankerungselement eine Sakral-Abstützplatte (50) und/oder eine Illio-Sakral-Abstützplatte (150) mit einer generell länglichen Form aufweist, die in ihrem mittleren Teil den zylindrischen Gewindeteil (5) aufweist, welcher von der Abstützfläche mit der ersten rutschfesten Erhebung (55) gesäumt ist, die in der Nähe eines ersten Endes (45) einen Bereich mit einer verstärkten Dicke aufweist, der von einer Bohrung (46) für den Durchgang einer Standard-Knochenschraube durchdrungen ist, und die in der Nähe ihres zweiten Endes (47) zwei andere Bohrungen (48, 49), für den Durchgang einer Standard-Knochenschraube mit geneigter Achse aufweist.

8. Vorrichtung nach einem der Ansprüche 1 bis 7, dadurch gekennzeichnet, daß sie als Verankerungselement eine Abstützplatte (56) auf dem Körper eines Wirbels aufweist, die oval und gekrümmt ausgebildet ist und die den zylindrischen Gewindeteil (5) auf dem mittleren Bereich ihrer konvexen Fläche (58) mit einer rutschfesten Erhebung (59) aufweist sowie zu beiden Teilen dieses mittleren Bereichs zwei Bohrungen (60, 61) für den Durchgang von Knochenschrauben mit versetzten Achsen.

9. Vorrichtung nach einem der Ansprüche 1 bis 8, dadurch gekennzeichnet, daß sie ein Gleitstück (104) mit einer doppelten Verbindung aufweist, bei welchem wenigstens einer (20) der Schenkel (19, 20) des Verbindungsteils (18) zwei Bereiche (120, 220) aufweist, die durch einen quer verlaufenden bogenförmigen Ausschnitt (320) voneinander getrennt und jeweils mit Durchgangslöchern (420, 520) für eine Festklemmschraube versehen sind, die mit Gewindelöchern (119, 219) korrespondieren, welche an dem anderen Schenkel (19) vorgesehen sind.

10. Vorrichtung nach einem der Ansprüche 1 bis 9, dadurch gekennzeichnet, daß sie wenigstens ein Verbindung-Gleitstück (4) von solcher Art aufweist, daß
- das Verbindung-Gleitstück (4) zwei generell flache Schenkel (19, 20) aufweist, die voneinander getrennt sind und sich jeweils an einen generell zylindrischen Festklemmteil (17) anschließen, der sie miteinander verbindet,
- der erste Verbindungsschenkel (19), der die erste Berührungsfläche (25) aufweist, die für eine Anlage an die Abstützfläche (12) des Verankerungselements bestimmt ist, nach außen in Bezug auf die Achse (II-II) des Festklemmteils (17) versetzt ist.

11. Vorrichtung nach Ansprüche 10, dadurch gekennzeichnet, daß
- die Verbindungsschenkel (19, 20) des Gleitstückes (4) jeweils ein einziges Durchgangsloch (23, 124) aufweisen,
- das Verankerungselement eine Doppelgewinde-Knochenschraube (1) nach Anspruch 5 ist, welche gleichzeitig die Funktion eines Festklemmelements und eines Verankerungselements erfüllt.

12. Vorrichtung nach einem der Ansprüche 1 bis 9, dadurch gekennzeichnet, daß das Verbindung-Gleitstück (105) generell die Form eines Parallelepipeds aufweist, welches axial mit einem Schlitz (28) geschlitzt ist, der einen ersten Bereich (29) mit einem kreisförmigen Querschnitt für den Durchgang des Versteifungsschaftes (3) und einen zweiten Bereich (30) mit einem verringerten Querschnitt aufweist, der in einer Verbreiterung (31) endet, wobei die Öffnung (32) des Schlitzes die seitliche Einführung des Versteifungsschaftes (3) erlaubt, wenn das Festklemmelement gelöst wird.

13. Vorrichtung nach Anspruch 10, dadurch gekennzeichnet, daß
- der erste Verbindungsschenkel (19), der die erste Berührungsfläche (25) aufweist, mit einer seitlichen (34) oder axialen (37) Verlängerung versehen ist, die nicht von dem zweiten Verbindungsschenkel (20) bedeckt und mit einem Durchgangsloch (35) für eine Gefäßstiel-Schraube versehen ist,
- der zweite Verbindungsschenkel (20) ein glattes Loch (124) für den Durchgang und den Einbau des Kopfes einer Festklemmschraube aufweist, deren Schaft sich in einem korrespondierenden Gewindeloch (23) des ersten Verbindungsschenkels (19) verschraubt.

14. Vorrichtung nach einem der Ansprüche 1 bis 13, dadurch gekennzeichnet, daß sie eine Anordnung von zwei gegenüberliegenden Haken (200, 202) aufweist, die mit entsprechenden Gleitstücken (4) verbunden sind, welche auf einem gemeinsamen Versteifungsschaft (3) angeordnet sind und von welchen der eine für eine Abstützung an der Lamina und der andere für eine Abstützung an dem Gefäßstiel eines gleichen Wirbels angepaßt ist.

15. Vorrichtung nach einem der Ansprüche 1 bis 14, dadurch gekennzeichnet, daß sie besteht aus:
- zwei Versteifungsschäften (3) mit ihren Verankerungselementen (1) und ihren entsprechenden Gleitstücken (4),
- wenigstens einem Steg (62), der mit jedem Versteifungsschaft (3) durch entsprechende Gleitstücke (4) verbunden ist, an welchen er über Flächen (68, 69) mit rutschfesten ersten Erhebungen (70, 71) anliegt.
